(19) Europäisches Patentamt
European Patent Office
Office européen des brevets

(11) Veröffentlichungsnummer: **0 179 931**
**B1**

(12) **EUROPÄISCHE PATENTSCHRIFT**

(45) Veröffentlichungstag der Patentschrift: **07.10.87**

(51) Int. Cl.⁴: **C 07 C 155/10,** C 07 D 295/20

(21) Anmeldenummer: **84112963.8**

(22) Anmeldetag: **27.10.84**

(54) **Verfahren zur Herstellung von Thiurampolysulfiden.**

(43) Veröffentlichungstag der Anmeldung: **07.05.86 Patentblatt 86/19**

(45) Bekanntmachung des Hinweises auf die Patenterteilung: **07.10.87 Patentblatt 87/41**

(84) Benannte Vertragsstaaten: **BE DE FR GB IT**

(56) Entgegenhaltungen:
**DE - A - 2 725 166**
**GB - A - 2 093 028**
**US - A - 2 794 021**

(73) Patentinhaber: **Akzo GmbH, Postfach 10 01 49 Kasinostrasse 19-23, D-5600 Wuppertal-1 (DE)**

(72) Erfinder: **Bergfeld, Manfred, Dr. Dipl.-Chem., August Pfeffer Strasse 4, D-8765 Erlenbach (DE)**
Erfinder: **Eisenhuth, Ludwig, Dr. Dipl.-Chem., Lautenhofstrasse 44, D-8753 Obernburg (DE)**
Erfinder: **Zengel, Hans-Georg, Dr. Dipl.-Chem., Nordring 6, D-8751 Kleinwallstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein Verfahren zur Herstellung von Thiurampolysulfiden aus sekundären Aminen, Schwefelkohlenstoff und Schwefel in Gegenwart eines Oxidationsmittels.

Thiurampolysulfide werden vor allem als Schwefeldonatoren und Beschleuniger bei der Vulkanisation von Kautschuk verwendet. Diese Substanzen werden z.B. unter dem Namen Thiuramtetrasulfid (Tetramethyl- oder Dipentamethylenthiuramtetrasulfid) oder Thiuramhexasulfid (Dipentamethylenthiuramhexasulfid) in den Handel gebracht. Die chemische Konstitution dieser Produkte ist bislang nicht genau bekannt, da Analysenmethoden zur Unterscheidung zwischen Mischungen verschiedener Polysulfide und Mischungen aus Polysulfiden und Schwefel fehlen. Ausserdem handelt es sich bei Thiurampolsulfiden um empfindliche Substanzen, die – besonders in gelöster Form – zur Abspaltung von Schwefel neigen. Diese Problematik wird z.B. in der DE-A-27 25 166 am Beispiel des als Tetramethylthiuramtetrasulfid bezeichneten Materials ausführlich beschrieben. Durch den Einsatz moderner Analysenmethoden wie z.B. Hochdruckflüssigkeitschromatographie (HPLC) und Gelpermeationschromatographie (GPC) kann man qualitativ aufzeigen, dass bei den oben genannten Handelsprodukten keine einheitlichen Verbindungen vorliegen, sondern Gemische aus mehreren Polysulfiden und freien Schwefel mit je nach Herstellungsmethode variierender quantitativer Zusammensetzung.

Im allgemeinen gehen die Verfahren zur Herstellung von Thiurampolysulfiden von den entsprechenden Dithiocarbamaten aus, die üblicherweise aus einem sekundären Amin, Schwefelkohlenstoff und einem Alkali- oder Erdalkalihydroxid hergestellt werden.

So beschreiben die US-PS 1 681 717 und 1 780 545 ein Verfahren zur Herstellung von Thiurampolysulfiden durch Umsetzung von Dithiocarbamaten mit Schwefelchloriden nach folgender Reaktionsgleichung:

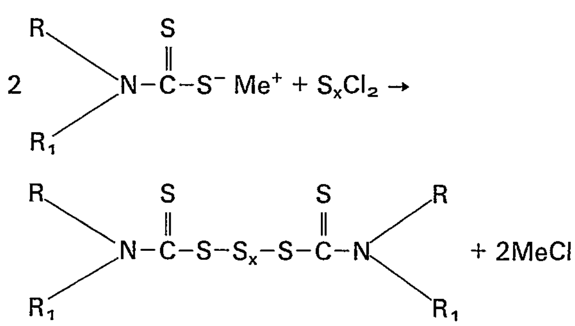

Hierbei werden jedoch nur geringe Ausbeuten erzielt. Ein verbessertes Verfahren zur Herstellung von Thiuramtetrasulfiden, insbesondere Dipentamethylenthiuramtetrasulfid, auf Basis obiger Reaktionsgleichung unter Verwendung von Schwefelmonochlorid wird in der US-PS 2 414 014 beschrieben. Mit diesem Verfahren können bis zu 95% Ausbeute erzielt werden.

Alle diese Verfahren haben jedoch die Nachteile gemeinsam, dass mit den korrosiven, übelriechenden Schwefelchloriden gearbeitet werden muss, und dass zusätzlich grosse Mengen unverwertbarer Salze als problematische Beiprodukte gebildet werden.

Ein Verfahren, das die zusätzliche Bildung von Natriumchlorid ausschliesst, beschreibt die DE-A-27 25 166. Nach diesem Verfahren wird Dimethylammoniumdimethyldithiocarbamat mit Wasserstoffperoxid in Gegenwart von Schwefelkohlenstoff und Schwefel zum Tetramethylthiuramtetrasulfid umgesetzt.

In einer Variante dieses Verfahrens gemäss DE-A-27 25 166 wird das umzusetzende Dithiocarbamatsalz in einem vorgeschalteten Reaktionsschritt aus Dimethylamin und Schwefelkohlenstoff in Wasser gebildet, und es kann anschliessend die hierbei erhaltene wässerige Lösung des Dimethylammoniumdimethyldithiocarbamats im selben Reaktionsgefäss mit Schwefel und Wasserstoffperoxid weiter zum Tetramethylthiuramtetrasulfid umgesetzt werden. So wird gemäss Beispiel 1 der DE-A-27 25 166 ein Reaktionsgefäss mit Wasser, Dimethylamin und 2 Tropfen eines nichtionischen grenzflächenaktiven Mittels gefüllt, die Lösung bei 25° C gerührt und Schwefelkohlenstoff während einer Zeitspanne von 14 Minuten zugesetzt, wobei die Temperatur auf 35° C steigt. Der Schwefel wird in einer Portion zugesetzt, woran sich die Zugabe von Wasser anschliesst. Dann wird Schwefelkohlenstoff der erhaltenen Aufschlämmung während einer Zeitspanne von 60 Minuten gleichzeitig mit Wasserstoffperoxid zugesetzt, wobei die Peroxidzugabe 2 Minuten nach Beginn der Schwefelkohlenstoffzugabe erfolgt. Das Endprodukt fällt schliesslich nach Filtration in einer Ausbeute von 90% an.

Dieses Verfahren stellt zwar eine Verbesserung gegenüber dem eingangs erwähnten Verfahren dar, ist allerdings in seiner Anwendung auf die Herstellung von Tetramethylthiuramtetrasulfid beschränkt. Weitere Nachteile sind das Erfordernis eines vergleichsweise teuren und wenig selektiven Oxidationsmittels (Wasserstoffperoxid), eines nichtionischen grenzflächenaktiven Mittels und die nicht quantitative Ausbeute.

Zur Herstellung von Thiuramdisulfiden wurde kürzlich ein weniger aufwendiges Verfahren durch Umsetzung von sekundären Aminen und Schwefelkohlenstoff in Gegenwart eines Oxidationsmittels in der deutschen Patentanmeldung P 31 05 622.9 beschrieben. Dieses Verfahren, das mit metallhaltigen Katalysatoren und Sauerstoff als Oxidationsmittel arbeitet, führt in hoher Ausbeute zu Thiuramdisulfiden.

Nach einem vereinfachten Verfahren zur Herstellung von Thiurampolysulfiden aus billigen Ausgangsverbindungen in hoher Ausbeute bestand weiterhin ein Bedürfnis.

Die hieraus resultierende Aufgabe konnte nun erfindungsgemäss gelöst werden durch ein Verfahren zur Herstellung von mit aliphatischen, araliphatischen und/oder cycloaliphatischen Kohlenwasserstoffresten substituierten Thiurampolysulfiden durch Umsetzung eines entsprechend substituierten sekundären Amins mit Schwefelkohlenstoff und Schwefel in einem Lösungsmittel und in Anwesenheit eines Oxidationsmittels, das dadurch gekennzeichnet ist, dass die Umsetzung mit einem sekundären Amin mit einem pKa-Wert $\geqslant 8$ bei Temperaturen von 0 bis 150°C durchgeführt wird und als Oxidationsmittel Sauerstoff oder ein sauerstoffhaltiges Gas und ein metallhaltiger Katalysator verwendet werden.

Das erfindungsgemässe Verfahren eignet sich für die Herstellung einer Vielzahl sehr unterschiedlich substituierter Thiurampolysulfide mit unterschiedlichem Schwefelgehalt.

Setzt man nur ein einziges sekundäres Amin als Reaktionspartner ein, so erhält man ein Thiurampolysulfid, welches an beiden Stickstoffatomen dieselben Substituenten trägt. Setzt man zwei unterschiedliche sekundäre Amine als Reaktionspartner ein, so kann man je nach Verfahrensbedingungen (Basizitätsunterschiede der Amine, Molverhältnisse etc.) Thiurampolysulfide mit zwei unterschiedlich substituierten Stickstoffatomen erhalten; als Nebenprodukte können hierbei noch mehr oder weniger grosse Mengen der beiden symmetrisch substituierten Thiurampolysulfide entstehen. Die Länge der Schwefelbrücke in den Thiurampolysulfiden wird durch die eingesetzte Schwefelmenge festgelegt. Setzt man z.B. 1 Grammatom Schwefel auf 1 Mol sekundäres Amin ein, so erhält man ein Produkt mit einer im Mittel aus 4 Schwefelatomen bestehenden Schwefelbrücke (Tetrasulfid). Setzt man 2 Grammatom Schwefel pro Mol Amin ein, so wird man im Mittel ein Hexasulfid erhalten.

Für das Verfahren sind alle sekundären Amine mit einem pKa $\geqslant 8$ geeignet. Ein derartiges sekundäres Amin wird dargestellt durch die Formel

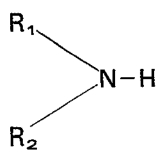

wobei $R_1$ und $R_2$ gleich oder verschieden sein können und jeweils $C_1$-$C_{18}$-Alkylreste wie z.B. Methyl, Äthyl, Propyl, n-Butyl, t-Butyl, Hexyl, Dodecyl und Octadecyl, Cycloalkylreste wie Cyclopentyl- und Cyclohexylrest und die durch Alkylgruppen substituierten Cyclopentyl- und Cyclohexylreste sowie durch Arylreste wie Phenyl und Naphthylreste substituierten $C_1$-$C_{18}$-Alkylreste darstellen. Die Substituenten des sekundären Amins können auch über ein gemeinsames Brückenglied miteinander verbunden sein. Beispiele für derartige Amine sind Piperidin, Pyrolidin und deren Derivate sowie andere Stickstoff-Heterocyclen.

Als Oxidationsmittel wird beim erfindungsgemässen Verfahren Sauerstoff oder ein sauerstoffhaltiges Gas, insbesondere Luft, verwendet.

Der Schwefel kann fest, flüssig oder gelöst, z.B. in Schwefelkohlenstoff, zugegeben werden. Dabei wurde festgestellt, dass es sich bei dem erfindungsgemässen Verfahren um eine völlig neuartige Reaktion handelt. Überraschenderweise wird nämlich bei dieser Reaktion eine wesentlich höhere Oxidationsgeschwindigkeit erzielt, als bei einer entsprechenden Reaktion ohne Schwefelzusatz, die zu einfachen Thiuramdisulfiden führt; das bedeutet, der Schwefel übt einen stark beschleunigenden Effekt auf die Oxidation aus.

Das Lösungsmittel ist beim erfindungsgemässen Verfahren nicht kritisch und es kommen so verschiedene Substanzklassen wie aromatische Kohlenwasserstoffe, wie Benzol, Toluol, Xylol, Nitrobenzol, aliphatische Ester, Alkyläther, niedere Alkohole wie Methanol, Äthanol, Isopropanol, n-Propanol, n-Butanol, t-Butanol und Amylalkohol, weiter Chlorkohlenwasserstoffe, wie Dichlormethan, Chloroform, Dichloräthan, Trichloräthan, ferner aprotische Lösungsmittel wie Dimethylformamid, Acetonitril, Dimethylacetamid, Dimethylsulfoxid und Hexamethylphosphorsäuretriamid sowie Wasser oder Gemische der genannten Lösungsmittel in Betracht.

Je nach eingesetztem sekundärem Amin können in reinem Wasser in einzelnen Fällen hohe Ausbeuten und Selektivitäten erzielt werden. Im allgemeinen ist die Reaktionsgeschwindigkeit in Wasser jedoch geringer als in den o.g. nichtwässerigen Lösungsmitteln. Vorzugsweise werden als Lösungsmittel aromatische Kohlenwasserstoffe, niedere Alkohole mit bis zu 6 C-Atomen, Gemische dieser Lösungsmittel oder Gemische dieser niederen Alkohole mit Wasser eingesetzt.

Als metallhaltige Katalysatoren eignen sich alle Metalle der Nebengruppen und deren Derivate, die zu einem leichten Valenzwechsel befähigt sind. Vorzugsweise werden als metallhaltige Katalysatoren Cer, Mangan, Kupfer, Eisen, Kobalt, Molybdän oder Vanadin in elementarer Form oder als Salze, Oxide, Komplexe oder in Form ihrer organischen Verbindungen eingesetzt. Von den bevorzugten Metallen bzw. deren Derivaten besitzen Kupfer, Mangan und Cer, verglichen mit Eisen, Kobalt, Molybdän und Vanadin die höhere katalytische Wirksamkeit, jedoch sind auch die letztgenannten Metalle und ihre Derivate ausgezeichnete Katalysatoren für die Oxydation.

Elementares Kupfer kommt vorzugsweise als Kupferpulver zur Anwendung. Als Kupferverbindung werden alle ein- oder zweiwertigen anorganischen, organischen, einfachen oder komplexen Kupfersalze in Betracht gezogen. Beispiele geeigneter einwertiger Kupfersalze sind Kupfer-(I)-chlorid, -bromid und -jodid, Additionsverbindungen dieser Kupfer-(I)-halogenide mit Kohlenmonoxid, komplexe Kupfer-(I)-salze, wie die Alkalichlorcuprate, komplexe Ammoniakate des Kupfer-(I)-cyanids, z.B. Cyanocuprate, wie Kaliumtricyanocuprat-(I), Doppelsalze mit Kupfer-(I)-rhodanid, Kupfer-(I)-acetat, Kupfer-(I)-sulfid und komplexe Doppelsulfide aus Kupfer-(I)-sulfid und Alkalipolysulfiden. Beispiele geeigneter Kupfer-(II)-salze sind Kupfer-(II)-chlorid, -bromid, -sulfid, -sulfat, -nitrat, -nitrit, -rhodanid, -cyanid, Cu-(II)-Salze von Carbonsäuren, wie Kupfer-(II)-acetat, Kupferdithiocarbamat sowie die

komplexen Ammoniakate von Kupfer-(II)-salzen. Auch Kupfer-(I)-oxid ist sehr gut als Katalysator geeignet.

Beispiele geeigneter manganhaltiger Katalysatoren sind Manganpulver, Mangandioxid, Kaliumpermanganate, Manganacetat und die Mangandithiocarbamate sowie die übrigen den obengenannten Kupferverbindungen entsprechenden Manganderivate. Als Beispiele geeigneter Cer-Katalysatoren werden metallisches Cer, Cerdioxid, Cer-(III)-chlorid, Cer-(IV)-chlorid und die Cerchlorokomplexsalze, Cernitrat und -nitratosalze, Cersulfat, Cercarbonat, Ceroxylat und die Cersulfide genannt.

Beispiele für Eisenkatalysatoren sind die bekannten Eisenoxide, Eisen-(II)- und Eisen-(III)-salze sowie die Komplexsalze.

Beispiele geeigneter Vanadinkatalysatoren sind die Vanadinoxide, -chloride und -sulfate sowie die bekannten Doppel- und Komplexsalze.

Geeignete Kobaltkatalysatoren sind die bekannten Kobaltoxide, Kobalt-(II)-salze und die Komplexsalze.

Schliesslich seien als Beispiele geeigneter Molybdänkatalysatoren die Oxide, Chloride, Sulfide und Fluoride, ferner die Molybdate sowie die bekannten komplexen Acidosalze genannt.

Selbstverständlich können auch Gemische aus mehreren der genannten Katalysatoren eingesetzt werden.

Die erforderliche Menge des metallhaltigen Katalysators ist überraschend gering. Sie liegt vorzugsweise im Bereich von 0,01 bis 5 mMol, bezogen auf 1 Mol sekundäres Amin. Es können auch geringere Katalysatormengen zur Anwendung gelangen, jedoch müssen hierbei längere Reaktionszeiten in Kauf genommen werden. Höhere Mengen an Katalysatoren sind nicht empfehlenswert, weil dann die Gefahr besteht, dass der Katalysator ausfällt und das Reaktionsprodukt verunreinigt.

Das erfindungsgemässe Verfahren wird bei Temperaturen im Bereich von 0 bis 150°C durchgeführt, vorzugsweise bei 20 bis 90°C. Temperaturen oberhalb 90°C erhöhen zwar die Raum/Zeitausbeute, werden jedoch aus sicherheitstechnischen Gründen weniger bevorzugt.

Vorzugsweise wird das erfindungsgemässe Verfahren bei Sauerstoffdrücken bzw. Sauerstoffpartialdrücken von wenigstens 0,1 bar durchgeführt. Wie zu erwarten, erhöht sich mit steigendem Druck die Reaktionsgeschwindigkeit. Aus sicherheitstechnischen Gründen wird ein Druckbereich von 1-10 bar bevorzugt.

Zur Durchführung des Verfahrens können die Reaktanten, Katalysator und Lösungsmittel in jeder beliebigen Reihenfolge zusammengegeben werden. Das sekundäre Amin und der Schwefelkohlenstoff werden dabei im allgemeinen ungefähr in stöchiometrischem Verhältnis eingesetzt (1:1); vorzugsweise wird der Schwefelkohlenstoff in einem leichten Überschuss (0,01 bis 0,2 Mol Überschuss) verwendet. Die einzusetzende Schwefelmenge kann je nach gewünschtem Endprodukt in weitem Bereich variiert werden. Bevorzugt wird ein Anteil von 1 bis 3 Grammatom Schwefel/Mol sekundäres Amin. Setzt man 1 Grammatom Schwefel pro Mol sekundäres Amin ein, so wird man im wesentlichen ein Thiuramtetrasulfid erhalten, bei 2 Grammatom Schwefel ein Thiuramhexasulfid, und bei noch grösseren Schwefelmengen werden entsprechend höhere Thiurampolysulfide gebildet. Besonders bevorzugt werden 1 bis 2 Grammatom Schwefel pro Mol sekundäres Amin eingesetzt.

Gemäss einer Ausführungsform der Erfindung werden das sekundäre Amin, der Schwefelkohlenstoff, der Schwefel und der metallhaltige Katalysator im Lösungsmittel aufgelöst bzw. suspendiert und in Anwesenheit von Sauerstoff bzw. eines sauerstoffhaltigen Gases zum entsprechenden Thiurampolysulfid umgesetzt. Ebensogut ist es möglich, zunächst nur den Schwefelkohlenstoff mit dem sekundären Amin (Molverhältnis 0,9 bis 1,1:2,0-2,2) umzusetzen und das erhaltene Reaktionsgemisch anschliessend mit Schwefel und Schwefelkohlenstoff (1,0-1,2 Mol) in Anwesenheit des metallhaltigen Katalysators und von Sauerstoff bzw. des sauerstoffhaltigen Gases umzusetzen. Ferner ist es möglich, das aus sekundärem Amin und Schwefelkohlenstoff als Zwischenprodukt gebildete Dithiocarbamat zu isolieren und anschliessend dieses Dithiocarbamat mit Schwefelkohlenstoff (Molverhältnis 1,0:1,0 bis 1,2) und Schwefel in Anwesenheit von Sauerstoff bzw. eines sauerstoffhaltigen Gases und des metallhaltigen Katalysators umzusetzen. Weiterhin ist es auch möglich, die Reaktionspartner sekundäres Amin, Schwefelkohlenstoff und Schwefel während der Reaktion der Reaktionslösung zuzuführen.

Die Reaktionsdauer hängt von den Verfahrensbedingungen sowie von dem eingesetzten sekundären Amin ab; im allgemeinen liegt sie im Bereich von wenigen Minuten bis zu mehreren Stunden. Unter günstigen Bedingungen bezüglich Temperatur und Sauerstoffdruck beträgt sie wenige Minuten bis 1 Stunde.

Die Durchführung des erfindungsgemässen Verfahrens erfolgt in einfacher Weise dadurch, dass der Sauerstoff bzw. das sauerstoffhaltige Gas unter den angegebenen Druck- und Temperaturbedingungen auf das Reaktionsgemisch aufgepresst oder in bzw. durch das Reaktionsgemisch geleitet wird. Das Ende der Reaktion (bei vollständigem Umsatz) lässt sich in einfacher Weise z.B. am Ende der Sauerstoffaufnahme erkennen.

In den meisten Fällen, wie z.B. bei den Tetramethylthiurampolysulfiden oder Dipentamethylenthiurampolysulfiden, fällt das Endprodukt sofort aus dem Reaktionsgemisch aus und kann abfiltriert werden. In anderen Fällen erhält man das gewünschte Produkt durch Abkühlen oder Einengen des Reaktionsgemisches. Flüssige Produkte werden durch destillative oder extraktive Aufarbeitung in reiner Form erhalten.

Bei der technischen Durchführung des erfindungsgemässen Verfahrens ist es vorteilhaft, die Mutterlauge im Kreislauf zu führen, wobei es nicht erforderlich ist, stets frischen metallhaltigen Katalysator zuzusetzen. Beispielsweise können mehr

als zehn Reaktionszyklen mit unverändert hoher Ausbeute durchgeführt werden, ohne dass ein Verlust an Katalysatoraktivität festgestellt wird.

Beim erfindungsgemässen Verfahren können in den meisten Fällen praktisch quantitative Ausbeuten und Selektivitäten von mehr als 99% erzielt werden. Die Produkte fallen in hoher Reinheit an und können in der Regel ohne Reinigung ihrer Bestimmung zugeführt werden. Bei entsprechendem Schwefelzusatz entsprechen die erhaltenen Produkte in ihrer chemischen Zusammensetzung den im Handel befindlichen Produkten (z.B. Tetramethyl- oder Dipentamethylenthiuramtetrasulfid oder Dipentamethylenthiuramhexasulfid).

Gegenüber dem bekannten zweistufigen Verfahren bei welchem zunächst die Dithiocarbamate synthetisiert werden, zeichnet sich das einstufige Verfahren durch seine Wirtschaftlichkeit und Umweltfreundlichkeit aus, da keine Hilfsstoffe verbraucht werden. Gegenüber dem aus der DE-A-27 25 166 bekannten einstufigen, aber lediglich auf die Herstellung von Tetramethylthiuramtetrasulfid beschränkten Verfahren besitzt das erfindungsgemässe Verfahren den Vorteil, dass in einfacher Reaktionsführung ein wesentlich preiswerteres Oxidationsmittel eingesetzt werden kann und dass praktisch quantitative Ausbeuten und hohe Selektivitäten erzielt werden.

Die folgenden Beispiele erläutern die Erfindung näher.

*Beispiel 1:*

In einem 1 l Glasautoklaven, der mit einem Doppelamantel zur Zirkulation einer Heizflüssigkeit, einem Thermometer, einem Druckmessgerät und einer Rührvorrichtung ausgerüstet ist, werden zu einer Lösung von 34,06 g (0,4 mol) Piperidin und 6,1 mg ($0,025 \cdot 10^{-3}$ mol) Mangan-II-acetat-tetrahydrat in 300 ml Methanol, 25,64 g (0,8 Grammatom) Schwefel und 31,2 g (0,41 mol) Schwefelkohlenstoff gegeben. Das Reaktionsgemisch wird auf 50°C erwärmt, intensiv gerührt und mit 1,7 bar Sauerstoff beaufschlagt. Sofort wird ein Sauerstoffverbrauch registriert, und es bildet sich ein fast weisser, feiner Niederschlag. Nach 30 min ist die Reaktion beendet (keine weitere Sauerstoffaufnahme, Farbumschlag der Reaktionslösung von braun nach blassgelb). Das Piperidin ist vollständig umgesetzt. Der gebildete Niederschlag wird abfiltriert, mit Methanol gewaschen und getrocknet. Es fallen 88,7 g Produkt mit einem Schmelzbereich von 126-129°C an.

Das Produkt entspricht in seiner Zusammensetzung dem Dipentamethylenthiuramhexasulfid.

Analyse: Dipentamethylenthiuramhexasulfid $C_{12}H_{20}N_2S_8$:

| | | | | |
|---|---|---|---|---|
| ber.: | C 32,11 | H 4,49 | N 6,24 | S 57,15% |
| gef.: | C 32,3 | H 4,5 | N 6,1 | S 56,8 % |

Durch Hochdruckflüssigkeitschromatographie-Analyse wird nachgewiesen, dass der Gehalt an freiem Schwefel im Produkt und der Gehalt an Dipentamethylenthiuramdisulfid jeweils unter 1% liegen.

Die Mutterlauge enthält noch weitere 0,7 g des Produktes, die durch Einengen oder starkes Abkühlen isoliert werden können. Demnach beträgt die Gesamtausbeute 89,4 g, entsprechend 99,5% d. Th. Das nach diesem Verfahren hergestellte Produkt entspricht als Schwefeldonator den im Handel befindlichen, als Dipentamethylenthiuramtetrasulfid bezeichneten Produkten.

*Beispiel 2 (Vergleichsbeispiel):*

Es wird wie in Beispiel 1 gearbeitet, aber ohne Schwefelzusatz. Die Sauerstoffaufnahme erfolgt nun wesentlich langsamer. Nach 30 min Reaktionszeit werden lediglich 16,2 g Dipentamethylenthiuramdisulfid (entsprechend 25% d. Th.) mit einem Schmelzpunkt von 132°C gebildet. Das Beispiel zeigt, dass der Schwefel die Reaktion stark beschleunigt.

*Beispiel 3:*

Es wird wie in Beispiel 1 gearbeitet, aber nur 12,82 g (0,4 Grammatom) Schwefel verwendet. Die Reaktionszeit beträgt nun 65 min. Dabei wird ein Produkt mit einem Schmelzbereich 124-127°C erhalten, das in seiner Zusammensetzung dem Dipentamethylenthiuramtetrasulfid entspricht.

Analyse: Dipentamethylenthiuramtetrasulfid $C_{12}H_{20}N_2S_6$:

| | | | | |
|---|---|---|---|---|
| ber.: | C 37,46 | H 5,24 | N 7,28 | S 50,1% |
| gef.: | C 37,8 | H 5,3 | N 7,3 | S 49,7% |

Der Gehalt an freiem Schwefel liegt unter 1% (Hochdruckflüssigkeitschromatographie-Analyse).

Die Ausbeute beträgt 88,9 g (99,0% d. Th.).

*Beispiel 4:*

In der in Beispiel 1 beschriebenen Weise werden 17,03 g (0,2 mol) Piperidin, 15,96 g (0,21 mol) Schwefelkohlenstoff und 19,2 g (0,6 Grammatom) Schwefel in 300 ml Methanol und bei Gegenwart von 6,1 mg ($0,025 \cdot 10^{-3}$ mol) Mangan-II-acetat und Sauerstoff umgesetzt. Die Reaktionstemperatur beträgt 50°C, der Sauerstoffdruck 1,7 bar und die Reaktionszeit 60 min.

Das so erhaltene Dipentamethylenthiurampolysulfid schmilzt in einem Temperaturbereich von 112-120°C und besitzt eine elementare Zusammensetzung von $C_{12}H_{20}N_2S_{10}$.

Die Ausbeute beträgt 49,83 g (97,2% d. Th.).

*Beispiele 5-8:*

Es wird wie in Beispiel 1 gearbeitet, jedoch werden verschiedene Lösungsmittel eingesetzt. Die Ergebnisse sind in Tabelle 1 zusammengefasst.

*(Tabelle auf der nächsten Seite)*

*Beispiele 9-12:*

Es wird wie in Beispiel 1 gearbeitet, jedoch mit anderen Katalysatoren und bei unterschiedlichen Reaktionstemperaturen. Die entsprechenden Reaktionszeiten sowie Produktausbeuten sind in Tabelle 2 wiedergegeben.

*Tabelle 1*

| Beispiel | Lösungsmittel (300 ml) | Reaktionszeit (min) | Ausbeute (% d. Th.) |
|---|---|---|---|
| 5 | Äthanol | 45 | 99,2 |
| 6 | Isopropanol | 65 | 99,5 |
| 7 | Toluol | 220 | 96,8 |
| 8 | Methanol/7,5% Wasser | 60 | 98,7 |

*Tabelle 2*

| Beispiel | Katalysator (mmol) | Reaktions-temp. (°C) | Reaktions-zeit (min) | Ausbeute (% d. Th.) |
|---|---|---|---|---|
| 9 | $Cu(OAc)_2$ $H_2O$ (0,1) | 50 | 120 | 96,5 |
| 10 | $CuSO_4$ (0,05) | 60 | 110 | 95,8 |
| 11 | $Ce(NO_3)_3$ (0,05) | 40 | 45 | 98,0 |
| 12 | $NH_4Ce(NO_3)_4$ (0,02) | 50 | 35 | 97,4 |

*Beispiel 13:*

Im folgenden Beispiel wird als sauerstoffhaltiges Gas Luft verwendet. In der in Beispiel 1 beschriebenen Weise werden 34,06 g (0,4 mol) Piperidin, 31,2 g (0,41 mol) Schwefelkohlenstoff und 25,64 g (0,8 Grammatom) Schwefel in 300 ml Methanol bei Gegenwart von 12,2 mg (0,05 mmol) Mangan-II-acetat und Luft (Gesamtdruck 5 bar) zur Reaktion gebracht. Die Reaktionstemperatur beträgt 60° C, die Reaktionszeit 40 min. Dabei wird das Dipentamethylenthiuramhexasulfidprodukt in einer Ausbeute von 88,1 g, entsprechend 98,3% d. Th. erhalten.

*Beispiele 14-17:*

In den folgenden Beispielen werden andere sekundäre Amine eingesetzt. Die Versuchsdurchführung erfolgt jeweils in der in Beispiel 1 beschriebenen Weise. Als Lösungsmittel wird jeweils 300 ml Methanol verwendet, der Sauerstoffdruck beträgt 1,7 bar.

Die weiteren Reaktionsbedingungen sowie die Ausbeuten und Schmelzpunkte der entsprechenden Thiurampolysulfide sind in Tabelle 3 zusammengefasst.

*Tabelle 3*

| Beispiel | sek. Amin (mol) | $Cs_2$ (mol) | Schwefel (Grammatom) | Mn-II-acetat (mmol) | Reaktionszeit (min.) | Reaktionstemp. (°C) | Produkt Fp. (°C) | Ausbeute (% d. Th.) |
|---|---|---|---|---|---|---|---|---|
| 14 | $(CH_3)_2NH$ (0,4) | 0,41 | 0,4 | 0,025 | 95 | 50 | $((CH_3)_2NCS_2)_2S_2$ 108-127 | 97,5 |
| 15 | $(CH_3)_2NH$ (0,4) | 0,41 | 0,8 | 0,025 | 50 | 50 | $((CH_3)_2NCS_2)_2S_4$ 99-104 | 98,3 |
| 16 | $(C_2H_5)NH$ (0,3) | 0,31 | 0,3 | 0,2 | 135 | 25 | $((C_2H_5)_2NCS_2)_2S_2$ wachsartig | 94,5 |
| 17 | $(CH_2)_4NH$ (0,4) | 0,41 | 0,8 | 0,25 | 70 | 50 | $((CH_2)_4NCS_2)_2S_4$ 134-136 | 93,6 |

**Patentansprüche**

1. Verfahren zur Herstellung von mit aliphatischen, araliphatischen und/oder cycloaliphatischen Kohlenwasserstoffresten substituierten Thiurampolysulfiden durch Umsetzung eines entsprechend substituierten sekundären Amins mit Schwefelkohlenstoff und Schwefel in einem Lösungsmittel und in Anwesenheit eines Oxidationsmittels, dadurch gekennzeichnet, dass die Umsetzung mit einem sekundären Amin mit einem pKa-Wert ⩾8 bei Temperaturen von 0 bis 150° C durchgeführt wird und als Oxidationsmittel Sauerstoff oder ein sauerstoffhaltiges Gas und ein metallhaltiger Katalysator verwendet werden.

2. Verfahren nach Anspruch 1, dadurch gekennzeichnet, dass pro Mol sekundäres Amin 1 bis 1,2 Mol Schwefelkohlenstoff eingesetzt werden.

3. Verfahren nach den Ansprüchen 1 und 2, dadurch gekennzeichnet, dass pro Mol sekundäres Amin 1 bis 3 Grammatom Schwefel eingesetzt werden.

4. Verfahren nach den Ansprüchen 1 bis 3, dadurch gekennzeichnet, dass der metallhaltige Katalysator in Mengen von 0,01 bis 5 mMol, bezogen auf ein Mol sekundäres Amin eingesetzt wird.

5. Verfahren nach den Ansprüchen 1 bis 4, dadurch gekennzeichnet, dass als Katalysator Cer, Mangan, Kupfer, Eisen, Kobalt, Molybdän oder Vanadin in elementarer Form, als Salze, Oxide, Komplexe oder organische Verbindungen oder ein Gemisch derselben eingesetzt wird.

6. Verfahren nach den Ansprüchen 1 bis 5, dadurch gekennzeichnet, dass als Lösungsmittel ein gegebenenfalls substituierter aromatischer Kohlenwasserstoff, ein niederer Alkohol mit bis zu 6 C-Atomen, ein Gemisch dieser Lösungsmittel oder ein Gemisch des niederen Alkohols mit Wasser eingesetzt wird.

7. Verfahren nach den Ansprüchen 1 bis 6, dadurch gekennzeichnet, dass die Reaktion bei Temperaturen von 20 bis 90° C durchgeführt wird.

## Claims

1. A process for the production of thiuram polysulfides substituted by aliphatic, araliphatic and/or cycloaliphatic hydrocarbon radicals by reaction of a correspondingly substituted secondary amine with carbon disulfide and sulfur in a solvent and in the presence of an oxidizing agent, characterized in that the reaction is carried out with a secondary amine having a pKa-value of ⩾ 8 at temperatures of from 0 to 150° C in the presence of oxygen or an oxygen-containing gas as oxidizing agent and a metal-containing catalyst.

2. A process as claimed in Claim 1, characterized in that from 1 to 1.2 moles carbon disulfide are used per mole secondary amine.

3. A process as claimed in Claims 1 and 2, characterized in that from 1 to 3 gram atoms sulfur are used per mole secondary amine.

4. A process as claimed in Claims 1 to 3, characterized in that the metal-containing catalyst is used in quantities of from 0.01 to 5 mmoles per mole secondary amine.

5. A process as claimed in Claims 1 to 4, characterized in that cerium, manganese, copper, iron, cobalt, molybdenum or vanadium is used as catalyst in elemental form, as salts, oxides or complexes or organic compounds or a mixture thereof.

6. A process as claimed in Claims 1 to 5, characterized in that the solvent used is an optionally substituted aromatic hydrocarbon, a lower alcohol containing up to 6 C-atoms, a mixture of these solvents or a mixture of the lower alcohol with water.

7. A process as claimed in Claims 1 to 6, characterized in that the reaction is carried out at temperatures of from 20 to 90° C.

## Revendications

1. Procédé de préparation de polysulfures de thiurame substitués avec des radicaux hydrocarbonés aliphatiques, araliphatiques et/ou cycloaliphatiques, par réaction d'une amine secondaire substituée de façon correspondante avec du sulfure de carbone et du soufre, dans un solvant et en présence d'un oxydant, *caractérisé* en ce que la réaction est effectuée à des températures comprises entre 0 et 150° C, avec une amine secondaire présentant une valeur de pKa ⩾ 8, et en ce que l'on utilise un catalyseur métallique et, comme oxydant, de l'oxygène ou un gaz contenant de l'oxygène.

2. Procédé conforme à la revendication 1, *caractérisé* en ce que l'on utilise de 1 à 1,2 mole de sulfure de carbone par mole d'amine secondaire.

3. Procédé conforme aux revendications 1 et 2, *caractérisé* en ce que l'on utilise de 1 à 3 atomegrammes de soufre par mole d'amine secondaire.

4. Procédé conforme aux revendications 1 à 3, *caractérisé* en ce que l'on utilise le catalyseur métallique en une quantité de 0,01 à 5 mmoles, par rapport à 1 mole d'amine secondaire.

5. Procédé conforme aux revendications 1 à 4, *caractérisé* en ce que l'on utilise comme catalyseur du cérium, du manganèse, du cuivre, du fer, du cobalt, du molybdène ou du vanadium, sous forme élémentaire ou sous forme de sels, d'oxydes, de complexes, ou de composés organiques, ou bien un mélange des mêmes.

6. Procédé conforme aux revendications 1 à 5, *caractérisé* en ce que l'on utilise comme solvant un hydrocarbure aromatique éventuellement substitué, un alcool inférieur comportant jusqu'à 6 atomes de carbone, un mélange de ces solvants ou un mélange d'un alcool inférieur avec de l'eau.

7. Procédé conforme aux revendications 1 à 6, *caractérisé* en ce que l'on effectue la réaction à des températures situées entre 20 et 90° C.